# EUROPÄISCHE PATENTANMELDUNG

(11) **EP 2 389 855 A1**
(43) Veröffentlichungstag der Anmeldung: **30.11.2011**
(21) Anmeldenummer: 11004529.1
(22) Anmeldetag: 11.03.2008
(51) Int. Cl.: A61B 1/24, A61B 1/05, A61B 1/247, G03B 15/03, G03B 17/17, A61B 1/00

(54) **Diagnosekamera sowie Aufsatz zur Realisierung einer solchen**

(30) Priorität: 16.03.2007 DE 102007013355
(62) Teilanmeldung aus: 08716438.0
(71) Anmelder: Dürr Dental AG, 74321 Bietigheim-Bissingen (DE)
(72) Erfinder: Die Erfindernennung liegt noch nicht vor
(74) Vertreter: Ostertag, Reinhard

(57) **Zusammenfassung**

Ein Aufsatz (40, 40a, 40b) für eine Diagnosekamera (10) umfasst einen zur lösbaren Festlegung an einem Kopfbereich (14) einer Diagnosekamera (10) gestalteten Koppelabschnitt (42) und einen mit diesem verbundenen formstabilen Abstandshalter (44), der einen freien Endabschnitt (46) aufweist.

## Beschreibung

Die Erfindung betrifft eine Diagnosekamera sowie einen Aufsatz zur Realisierung einer solchen.

Eine bekannte dentale Diagnosekamera ist für die Fernbetrachtung eines Mundraums eines Patienten vorgesehen. Ihr Gehäuse weist einen Greifabschnitt sowie einen damit verbundenen, schlanken Kopfbereich auf. Der Greifabschnitt ist zum Ergreifen und Führen der Diagnosekamera durch einen Benutzer vorgesehen. Der in Verlängerung des Greifabschnit-tes angebrachte Kopfbereich enthält eine Kameraeinheit mit optischen und elektronischen Komponenten wie eine Linsenoptik und einen Bildaufnehmer.

Mit der Kameraeinheit kann ein stark vergrößerbares Abbild des zu betrachtenden Mundraums oder der Zähne aufgenommen und in Form von elektrischen Signalen an ein Wiedergabegerät, beispielsweise einen Monitor, weitergegeben werden. Bedingt durch die Arbeitsbedingungen (gebückte Haltung, schlechter direkter Sichtkontakt zur Beobachtungsstelle) fallt es schwer, ein scharfes und ruhendes, nicht verwak-keltes Diagnosekamerabild des Mundraums bzw. der Zähne im Mundraum aufzunehmen.

Durch die vorliegende Erfindung soll ein Weg aufgezeigt werden, wie man einen ruhigeren Bildstand und gute Bild-^ schärfe bei einer Diagnosekamera erhält.

Diese Aufgabe ist erfindungsgemäß durch eine Diagnosekamera gemäß den Merkmalen des Anspruchs I und durch einen Diagnosekamera-Aufsatz mit den Merkmalen des Anspruchs 1 gelöst.

Die erfindungsgemäße Diagnosekamera weist zumindest einen formstabilen Abstandshalter mit einem freien Endabschnitt auf, der zumindest überwiegend außerhalb des Gesichtsfeldes angeordnet ist. Mittels des freien Endabschnittes kann ein durch die optischen Komponenten der Diagnosekamera bestimmter Objektbereich, also ein Lagebereich, innerhalb dessen ein Objekt durch die Diagnosekamera scharf abgebildet wird, für den Benutzer bei der Verwendung der Diagnosekamera sichtbar gemacht und/oder taktil erfahrbar gemacht werden.

Der Benutzer kann dadurch die Diagnosekamera in einem richtigen Abstand zu dem aufzunehmenden Objekt, insbe-sondere einem Zahn, im Mundraum positionieren, ohne dass er dazu den typischerweise dem Patienten zugewandten Monitor, auf dem das Bild des Objektes dargestellt wird, betrachten muss.

Der Abstandshalter ist vorzugsweise derart ausgebildet, dass sein freies Ende in der Objektebene der Diagnosekamera angeordnet ist.

Der freie Endabschnitt des Abstandshalters gibt auch eine Auflagefläche vor, die z.B. auf die Oberfläche des aufzunehmenden Zahns aufgesetzt werden kann, wodurch eine stabilisierung des von der Diagnosekamera aufgenommenen Diagnosekamerabilds erhalten wird, wobei das Objekt gleichzeitig in der Objektebene der Diagnosekamera liegt.

Vorteilhafte Weiterbildungen der Erfindung sind in den Unteransprüchen angegeben.

Bei einer Diagnosekamera nach Aspekt 3 ist der freie Abstandshalter als zumindest im Wesentlichen umlaufende Hülse gestaltet und gibt somit eine Auflagefläche auf. Dadurch kann eine Verkippung der Diagnosekamera gegenüber dem aufzunehmenden Objekt, insbesondere einem Zahn, durch flächige Auflage des Ausatzes auf dem Objekt gering gehalten werden. Dadurch werden Verzerrungen des Bildes verringert, und die Abbildungsqualität für das Objekt wird verbessert.

Bei einer besonders vorteilhaften Ausführungsform dieser Variante ist der freie Endabschnitt als geschlossene, umlaufende Wand ausgeführt, so dass eine Auflage der Diagnosekamera auf dem abzubildenden Objekt unabhängig von der Orientierung des Greifabschnittes des Kameragehäuses gewährleistet ist.

Die Weiterbildung der Erfindung gemäß Aspekt 4 ist im Hinblick auf eine zuverlässige Verbindung zwischen dem Ab-standshalter und der Diagnosekamera von Vorteil. Es wird so vermieden, dass sich der Abstandshalter während der Benutzung der Diagnosekamera vom Kopfbereich löst und in den Mundraum fällt.

Bei der Diagnosekamera nach Aspekt 5 kann eine vorteilhafte Ausrichtung der Endfläche des freien Endabschnittes in der von der Diagnosekamera vorgegebenen Objektebene verwirklicht werden. Typischerweise weist eine Diagnosekamera eine (vorzugsweise um 90 Grad) gegenüber einer Mittellängsachse des Greifabschnittes des Gehäuses gekippte Blickrichtung auf. Damit liegt der freie Endabschnitt des ebenfalls (vorzugsweise um 90 Grad) abgewinkelten Kanals, den Abstandhalter und Koppelabschnitt bilden, zumindest nahezu.in der Objektebene.

Die Weiterbildung der Erfindung nach Aspekt 6 gewährleistet, dass es nicht zu unerwünschten Reflexen an der Innenseite des Kanals im Bereich des Abstandshalters kommt, was die Bildqualität verschlechtern würde.

Die Innenseite des Kanals kann.gemäß Aspekt 7 gegenüber den ansonsten glatt ausgeführten Oberflächen des Aufsatzes eine vergrößerte Rauhigkeit aufweisen. Beispielsweise ist die Innenseite des Kanals mit einer gemittelten Rautiefe R_{z} von 1 um bis 40um, vorzugsweise von 2um bis 10 um, besonders bevorzugt weniger als 4 um, versehen. Ergänzend oder alternativ können auch Rillen an der Innenseite des Kanals vorgesehen sein, dir vorzugsweise in Umfangsrichtung, orthogonal zur Blickrichtung verlaufen. Die Rauhigkeit bewirkt eine diffuse Streuung auftreffender Lichtstrahlen und ermöglicht somit eine Reflexverminderung.

Der Diagnosekamera nach Aspekt 8 ermöglicht durch die vorzugsweise konische oder pyramidenförmige Erweiterung zum freien Ende hin eine kompakte Gestaltung des Abstandshal-t.ers, ohne dass dadurch das Gesichtsfeld der Diagnosekamera begrenzt wird.

Die Weiterbildung der Erfindung nach Aspekt 9 gewährleistet eine kostengünstige Herstellung sowie eine robuste Gestaltung des Abstandshalter-Aufsatzes, da der Koppelabschnitt und der Abstandshalter einstückig, insbesondere im Kunststoffspritzgussverfahren, hergestellt sind.

Vorzugsweise wird der Aufsatz gemäß Aspekt 10 aus einem sterilisierbaren Kunststoff, beispielsweise einem Polypropylen, hergestellt. Damit kann eine Wiederverwendbarkeit unter Einhaltung der für medizinische und zahnmedizinische Instrumente geltenden Desinfektions- und/oder Steriisie-rungsrichtlinien gewährleistet werden. Zudem kann die Diagnosekamera mit einem geringen Gewicht verwirklicht werden.

Der Diagnosekamera nach Aspekt 11 ist derart ausgeführt, dass das Kameragehäuse und der Koppelabschnitt zusammenarbeitende Rastmittel aufweisen. Damit kann in einfacher Weise eine lösbare Befestigung und eine zuverlässige Festlegung des Abstandshalter-Aufsatzes an der Diagnosekamera verwirklicht werden.

Ein Abstandshalter-Aufsatz gemäß Anspruch 1 und hiervon abhängenden Unteransprüchen erlaubt es, die obengenannten Vorteile bei schon im Feld befindlichen Diagnosekameras auch zu erhalten.

Gemäß der Erfindung wird somit (an Fabrik oder durch Nachrüstung) eine Diagnosekamera erhalten, die ein Auflegen und Abstützen der Diagnosekamera auf dem aufzunehmenden Objekt ermöglicht. Damit kann ein verwacklungsarmes bzw. verwacklungsfreies Bild des Objektes erzielt werden. Bei Ausführung aus Kunststoff wird neben einem günstigen Herstellungspreis ein sicherer, vorzugsweise formschlüssiger und damit präziser und sicherer Halt des Abstandshalter-Auf satzes am Kopfbereich der Diagnosekamera gewährleistet. Die Elastizitätseigenschaften des Kunststoffmaterials ermöglichen es, Verletzungen des Zahnfleisches oder der Mundschleimhaut auch bei dünnwandiger Gestaltung des Abstandshalter-Auf satzes zu vermeider, da keine harten Kanten auf dem Zahn aufliegen oder mit dem Zahnfleisch in Verbindung kommen.

Nachstehend wird die Erfindung anhand von Ausführungsbeispielen unter Bezugnahme auf die Zeichnungen näher erläutert. Dabei zeigen:
- Fig. 1: eine perspektivische Darstellung einer dentalen Diagnosekamera mit einem Greifabschnitt und einem Kopfbereich;
- Fig. 2: eine perspektivische Darstellung der dentalen Diagnosekamera nach Fig. 1 mit einem Abstandhalter-Aufsatz;
- Fig. 3: eine Schnittdarstellung des Kopfbereichs der en Diagnosekamera gemäß Fig. 2;
- Fig. 4: eine perspektivische Darstellung eines Abstandshalter-Aufsatzes mit geringer Baulänge;
- Fig. 5: eine perspektivische Darstellung eines Abstandshalter-Aufsatzes mit mittlerer Baulänge;
- Fig. 6: eine perspektivische Darstellung eines Abstandshalter-Aufsatzes mit großer Baulänge und einem pyramidenförmig aufgeweiteten Endbereich; und
- Fig. 7: eine seitliche Ansicht einer dentalen Diagnosekamera mit an das Kameragehäuse angeformtem Abstandhalter.

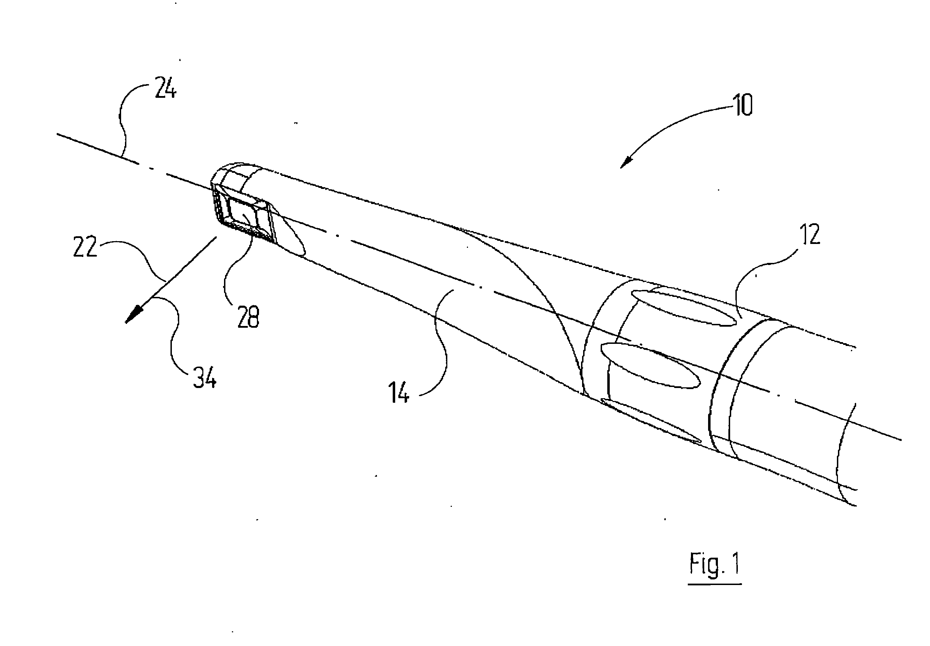

In der Fig. 1 ist eine dentale Diagnosekamera 10 dargestellt, die einen Greifabschnitt 12 und einen im Wesentlichen konusabschnittsförmig gestalteten, in Verlängerung des Greifabschnittes 12 angebrachten Kopfbereich 14 aufweist.

An einem vom Kopfbereich 14 abgewandten nicht dargestellten, hinteren Ende des Greifabschnittes 12 weist die dentale Diagnosekamera 10 ein nicht dargestelltes Kabel auf, das für die Bereitstellung elektrischer Energie und insbesondere für die Übermittlung der von der dentalen Diagnosekamera 10 erzeugten elektrischen Bildsignale vorgesehen ist. Die Bildsignale werden von einer in dem Kopfbereich 14 integrierten Kameraeinheit 16 erzeugt.

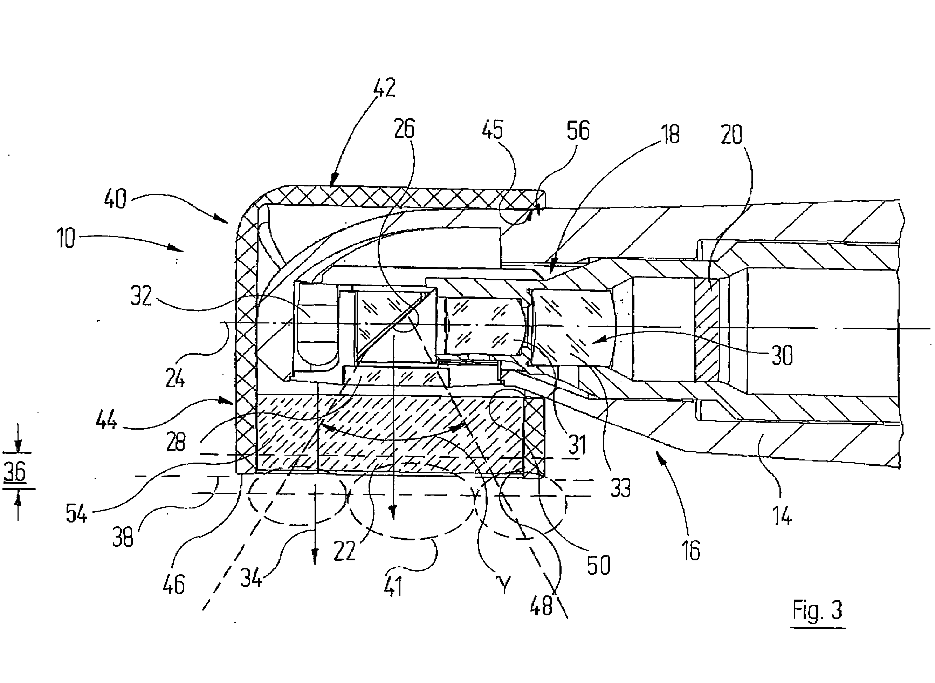

Die Kameraeinheit 16 besteht, wie in Fig. 3 näher dargestellt, im Wesentlichen aus einem optischen System 18 und einem Bildaufnehmer 20. Eine Blickrichtung 22 der Diagnosekamera 10 ist im Wesentlichen orthogonal zu einer Mittellängsachse 24 des Greifabschnittes 21 und des Kopfbereichs 14 ausgerichtet. Dadurch wird eine besonders ergonömische und für die Betrachtung von Zahnoberflächen im beengten Mundraum besonders vorteilhafte Handhabung der Diagnosekamera 10 gewährleistet.

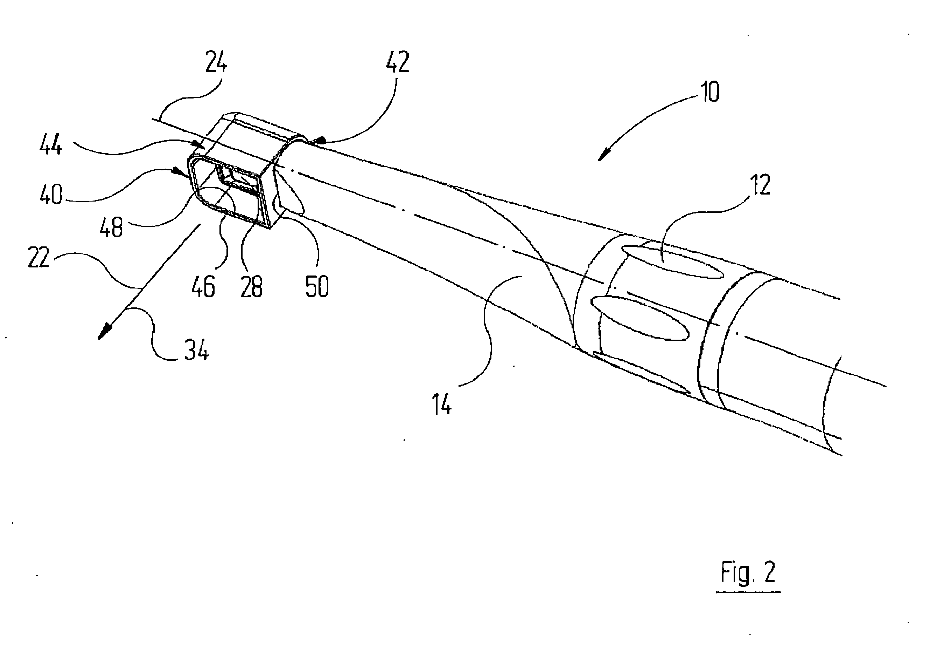

Gemäß der Fig. 2 ist auf die Diagnosekamera 10 ein Aufsatz 40 aufgesteckt, der das Einhalten eines bestimmten Abstandes zwischen der Diagnosekamera und dem zu betrachtenden Objekt erleichtert und der nachstehend anhand mehrerer Ausführungsbeispiele näher beschrieben wird.

Wie in der Schnittdarstellung der Fig. 3 näher dargestellt ist, werden von einem beleuchteten Objekt 41, beispielsweise einem Zahn in einem Mundraum eines Patienten, ausgehende Lichtstrahlen mittels des optischen Systems 18 auf einen Bildaufnehmer 20 abgebildet. Der Bildaufnehmer 20 ist z. B. ein CCD (Charge Coupled Device) und weist eine lichtempfindliche Sensorfläche auf, die pixelweise eine Umwandlung eingestrahlter Lichtstrahlen in elektrische Signale bewerkstelligt.

Die elektrischen Ausgangssignale des Bildaufnehmers 20 werden dann über das nicht dargestellte Kabel an eine ebenfalls nicht dargestellte Bildverarbeitungseinheit gegeben, die die Darstellung des Objektes 41 auf einem Monitor steuert.

Das optische System 18 weist ein dicht in die Wand der Kopfbereiches 16 eingesetztes transparentes Fenster 28, einen Umlenkspiegel 26 und ein Linsensystem 30 mit zwei Linsen 31, 33 auf. Das transparente Fenster 28 ist in den Kopfbereich 14 dicht eingeklebt, um die Kameraeinheit 16 in dem einen Teil des Kameragehäuses bildenden hülsenförmigen Kopfbereich 14 feuchtigkeitsgeschützt unterbringen zu können.

Der Umlenkspiegel 26 besorgt eine 90-Grad-Umlenkung der Lichtstrahlen, d.h. der vom Objekt 41 ausgehenden Objektstrahlen. Ein vom Objekt 41 ausgehender exemplarisch betrachteter Lichtstrahl kann nach Umlenkung durch den Umlenkspiegel 26 parallel zur Mittellängsachse 24 verlaufen und trifft senkrecht auf die Sensorfläche des Bildaufnehmers 20 auf. Die Blickrichtung 22 der Kameraeinheit 16 ist somit durch die Wirkung des Umlenkspiegels 26 orthogonal zur Mittellängsachse 24 der Diagnosekamera 10 ausgerichtet.

Der von dem Bildaufnehmer 20 erfassbare Bildwinkel γ wird im Wesentlichen von dem Linsensystem 30 bestimmt. Zudem bestimmt das Linsensystem 30 einen Tiefenschärfe- oder Objektbereich 36, innerhalb dessen ein Objekt scharf auf den Bildaufnehmer 20 abgebildet wird.

Innerhalb des Objektbereichs 36 kennzeichnet eine Objektebene 38 diejenige Ebene, in der ein Objekt maximal scharf abgebildet wird. Der Bildwinkel γ kann gegebenenfalls von der Größe des Fensters 28, von der Größe des Ümlenkspiegels 26 oder von einer im optischen Strahlengang vorgesehenen, nicht dargestellten Gesichtsfeldblende oder auch vom Aufsatz 40 begrenzt werden.

Benachbart zum Umlenkspiegel 26 ist eine als Weißlicht-Leuchtdiode ausgeführte Lichtquelle 32 vorgesehen, die der Beleuchtung des abzubildenden Objekts dient. Die Hauptabstrahlrichtung 34 der Lichtquelle 32 verläuft zumindest im Wesentlichen parallel zur Blickrichtung 22 der Kameraeinheit 16.

Der in der Schnittdarstellung der Fig. 3 gezeigte Aufsatz 40 ist endseitig auf den Kopfbereich 14 der Diagnosekamera 10 aufgeschoben und wird kraftschlüssig sowie formschlüssig am Kopfbereich 14 gehalten.

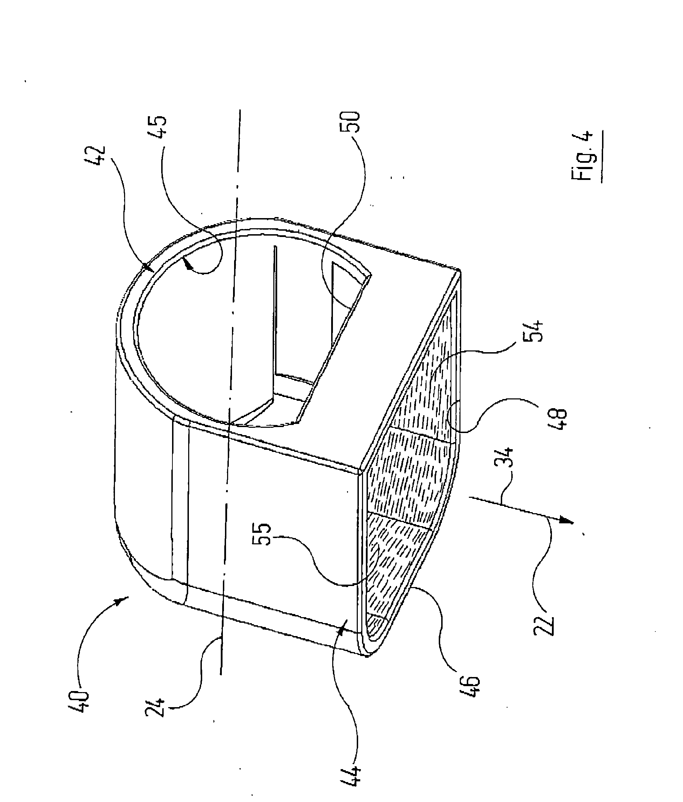

Der aus einem sterilisierbaren Kunststoff, beispielsweise Polypropylen, hergestellte Aufsatz 40 weist einen als Verbindungsmittel gestalteten Koppelabschnitt 42 sowie einen Abstandshalterabschnitt 44 auf. Wie in den Fig. 4 bis 6 näher dargestellt, begrenzen Wandabschnitte von Koppelabschnitt 42 und Abstandshalterabschnitt 44 eine Öffnung 45, in welche der Kopfbereich 16 formschlüssig einführbar ist..

Der Koppelabschnitt 42 und der Abstandshalterabschnitt 44 sind beide hülsenförmig und bilden zusammen einen rechtwinklig abgewinkelten Kanal. Die Länge des Kanals in Richtung der Mittellängsachse 24 ist bei den dargestellten Ausführungsformen des Aufsatzes 40 gemäß den Fig. 3 bis 6 recht kurz. Der Kanal kann bei einer nicht dargestellten weiteren Ausführungsform der Erfindung im Bereich des Koppelabschnitts auch bedeutend länger sein, um eine größere axiale Überdeckung mit dem Kopfbereich 16 zu erzielen.

Der Koppelabschnitt 42 ist hinsichtlich des Querschnitts der Öffnung 45 derart auf den Querschnitt des Kopfbereichs 14 abgestimmt, dass eine kraftschlüssige Kopplung des Aufsatzes 40 an den Kopfbereich 14 erhalten wird. Dazu ist der freie Innenquerschnitt des Koppelabschnitts 42 geringfügig kleiner als der Außenquerschnitt des Kopfbereichs 14 gewählt, so dass beim Aufstecken des Aufsatzes 40 auf den Kopfbereich 14 eine elastische Deformation des Koppelabschnitts 42 auftritt. Diese elastische Deformation stellt die für die gewünschte kraftschlüssige Festlegung des Aufsatzes 40 an der Diagnosekamera 10 notwendige Reibkraft bereit.

Zudem ist der Koppelabschnitt 42 mit einer nach innen ragenden Rastnase 56 versehen, die elastisch in eine im Kopfbereich 14 vorgesehene Nut eingreift und so eine zuverlässige Verrastung des Aufsatzes 40 an der Diagnosekamera 10 gewährleistet.

Der hülsenförmig gestaltete Abstandshalterabschnitt 44 weist einen in Blickrichtung 22 der Kameraeinheit 16 über das Fenster 28 überstehenden freien Endabschnitt 46 auf, der in der Objektebene 38 endet, dieser zumindest nahe benachbart ist.

Damit zeigt der freie Endabschnitt 46 dem Benutzer an, wie nahe die Diagnosekamera 10 an das Objekt 41 herangeführt werden muss, um ein scharfes Bild zu liefern. Wenn der freie Endabschnitt 46 auf eine Oberfläche eines Objekts 41 aufgesetzt wird, liegt die Objektoberfläche automatisch im Objektbereich 36 der Diagnosekamera 10.

Durch das Anliegen des freien Endabschnittes 46 an der Objektoberfläche wird zudem eine Lagestabilisierung der Diagnosekamera 10 erreicht. Dies erleichtert zum einen die Wahl des richtigen Bildausschnittes. Zum anderen werden Zitterbewegungen des Benutzers, die ohne ein Auflegen des freien Endabschnittes 46 auf das Objekt auftreten könnten, reduziert oder ganz vermieden. Damit ist das von der Diagnosekamera 10 aufgenommene Bild ruhig und stabil.

Der in der Fig. 4 dargestellte Aufsatz 40 weist einen kasten-ähnlichen Aufbau auf. Eine Seitenwand des Aufsatzes 40 ist vollständig ausgespart, wodurch ein von dem hülsenförmigen freien Endabschnitt 46 berandetes Sichtfenster 48 gebildet wird. An einer dem Sichtfenster 48 benachbarten Seitenwand des Aufsatzes 40 ist die Koppelzwecken dienende öffnung 45 vorgesehen, die eine im Wesentlichen kreisförmige Randkontur mit einer parallel zur Endfläche des Endabschnittes 46 ausgerichteten Abflachung 50 aufweist.

Der freie Endabschnitt 46 ist bei dem Aufsatz 40 gemäß der Fig. 4 ungefähr 5mm von der Abflachung 50 entfernt angeordnet, so dass der Abstand zwischen dem Objekt 41 und dem Fenster 28 ebenfalls ungefähr 5 mm beträgt und der freie Endabschnitt 46 zumindest nahezu in der Objektebene 38 liegt.

An einer Innenfläche des Aufsatzes 40 ist im Bereich des Abstandshalterabschnitts 44 eine umluafende aufgeraute Oberfläche 54 vorgesehen. Die Aufrauhung kann beispielsweise durch eine in der Spritzgussform für den Aufsatz 40 vorgesehene Oberflächenbearbeitung, beispielsweise durch Sandstrahlen, erzielt werden und wird beim Spritzvorgang auf den Aufsatz 40 abgeformt.

Die aufgeraute Oberfläche 54 bewirkt, dass von der Lichtquelle 32 oder vom Objekt ausgehende Lichtstrahlen an der Innenfläche des Abstandshalterabschnitts 44 diffus gestreut werden. Somit können diese Lichtstrahlen nicht als Streulichtstrahlen ungehindert in das optische System 18 der Kameraeinheit 16 eingestreut werden, wodurch sich eine Verbesserung der Bildqualität der Diagnosekamera 10 verwirklichen lässt.

Alternativ kann die Innenfläche des Abstandshalterabschnittes 44 Licht absorbierend, insbesondere schwarz ausgeführt werden, z. B. schwarz lackiert werden.

Durch die auf den Bildwinkel der Kameraeinheit abgestimmte Geometrie des Aufsatzes 40 wird zudem ein seitliches Entweichen von Lichtstrahlen, die von der Lichtquelle 32 abgestrahlt wurden, vermindert bzw. verhindert. Damit steht mehr Licht für die Beleuchtung des Objekts zur Verfügung, wodurch ein weiterer Beitrag für eine verbesserte Bildqualität erhalten wird.

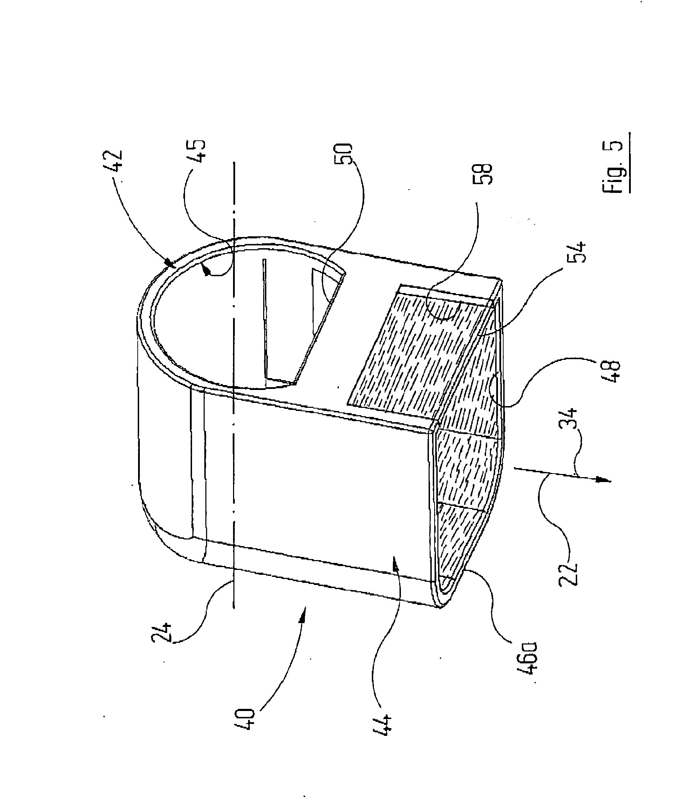

Bei dem in der Fig. 5 dargestellten Aufsatz 40, bei dem für funktionsgleiche Elemente die bereits eingeführten Bezugszeichen beibehalten werden, ist der freie Endabschnitt 46 ca. 10 mm von der Abflachung 50 entfernt angeordnet und ist durch eine Ausnehmung 58 teilweise unterbrochen.

Damit kann beim Auflegen der mit dem Aufsatz 40 versehenen Diagnosekamera 10 auf eine unebene Oberfläche gewährleistet werden, dass von der unebenen Oberfläche vorstehende Objektbereiche, die mittels der Diagnosekamera 10 dargestellt werden sollen, in der Objektebene 38 oder zumindest im Objektbereich 36 der Diagnosekamera 10 zu liegen kommen.

Zudem ermöglicht die Ausnehmung 58, die dem Benutzer der Diagnosekamera 10 zugewandt ist, einen direkten Blick auf die aufzunehmende Oberfläche.

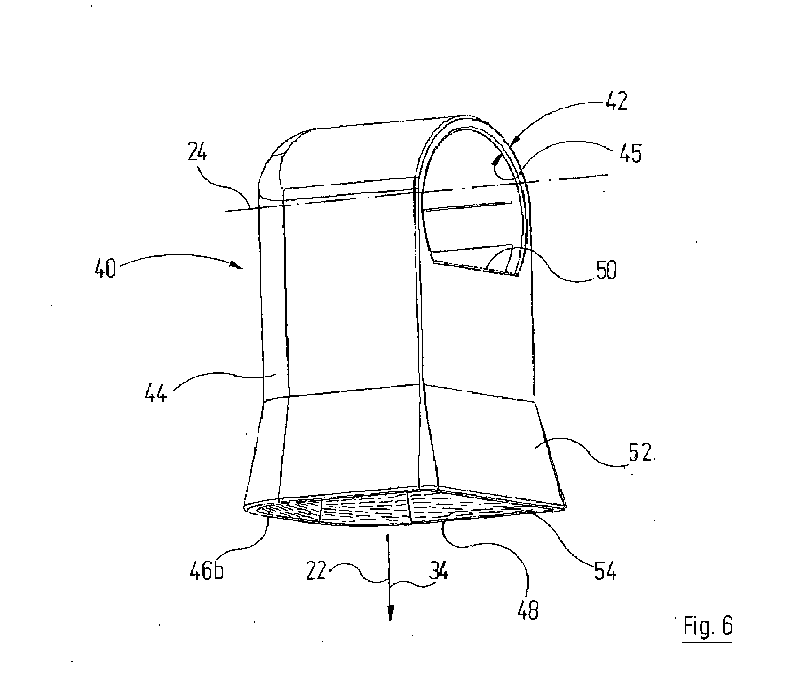

Bei dem in der Fig. 6 dargestellten Aufsatz 40, bei dem für funktionsgleiche Elemente die bereits eingeführten , Bezugszeichen beibehalten sind, ist der freie Endabschnitt 46 ca. 18mm von der Abflachung 50 entfernt angeordnet und befindet sich endseitig an einem im Wesentlichen pyramidenstumpfförmig gestalteten Fortsatz 52 des kastenförmigen Aufsatzes 40.

Mit der pyramidenstumpfförmigen Gestaltung des Fortsatzes 52 kann erreicht werden, dass der Aufsatz 40 kompakt baut und in einfacher Weise auf ein zu betrachtendes Objekt aufgesetzt werden kann. Der Öffnungswinkel des Fortsatzes 52, also der zwischen den jeweils gegenüberliegend angeordneten Pyramidenflächen eingeschlossene Winkel, ist derart auf den Bildwinkel γ der Kameraeinheit 16 angepasst, dass Randstrahlen, die noch vom Bildaufnehmer 20 erfasst werden können, im Wesentlichen parallel zu den Pyramidenflächen des Fortsatzes 52 verlaufen. Damit wirkt der Aufsatz 40 nicht als Begrenzung für das von der Kameraeinheit 16 aufgenommene Bild.

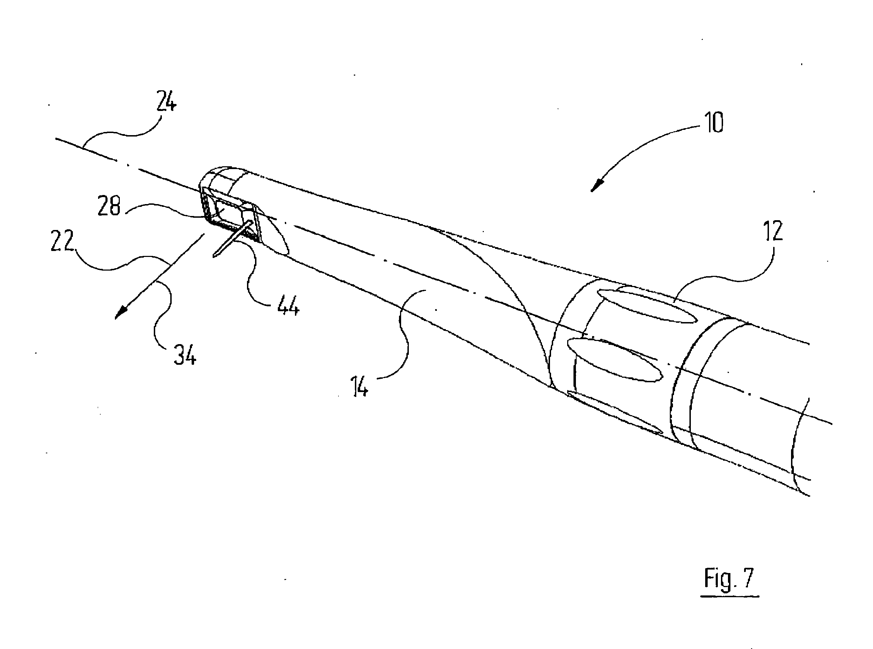

Beim Ausführungsbeispiel nach Figur 7 ist an den Kopfbereich 16 ein Abstandshalterstab 44 angeformt, dessen Achse senkrecht zur Achse von Greifabschnitt 14 und Kopfbereich 16 verläuft und seitlich außerhalb des Gesichtsfeldes liegt oder dem Rand des Gesichtsfeldes eng benachbart ist.

Alternativ kann der Abstandshalterstab 44 lösbar in eine Vertiefung des Kopfbereiches 16 eingesetzt sein, was im Hinblick auf Herstellung sowie Reinigung und Sterilisierung der Diagnosekamera vorteilhaft ist.

Man erhält so die gleichen Vorteile wie bei den Ausführungsbeispielen nach den Figuren 1 bis 6.

Die oben beschriebene Diagnosekamera kann auch zur Betrachtung anderer schlecht zugänglicher Körperteile von Mensch und Tier in der Humanmedizin oder der Vetrinärmedizin verwendet werden. Sie kann auch in der Werkstoff- und Produktprüfung zur Kontrolle schlecht zugänglicher Oberflächenbereiche eingesetzt werden.

Der freie Endabschnitt 46 des Abstandshalter 40 kann durch eine transparente Endplatte 55 dicht verschlossen sein, wie in Figur 4 angedeutet. Diese stellt eine Anlagefläche für zu betrachtendes Gewebe dar und sorgt so für eine präzise Positionierung desselben in der Objektebene. Zugleich werden Wölbungen des Gewebes verhindert. Auch ist so das freie Ende des Abstandshalters gegen Eindringen von Verunreinigungen und Keimen geschützt. Die glatte Außenfläche am Ende des Abstandhalters 40 erleichtert dessen Sterilisierung und Desinfektion.

Die Erfindung umfasst auch folgende weitere Aspekte:
1. Diagnosekamera, insbesondere für medizinische und dentale Zwecke, mit einem Gehäuse (14, 16), einer Optik (18) und einem Bildaufnehmer (20), die in dem Gehäuse (14, 16) untergebracht sind, bei der das Gehäuse (14, 16) objektseitig einen Abstandshalterabschnitt (44) trägt.
2. Diagnosekamera nach Aspekt 1, bei der der Abstandshalterabschnitt (44) auf einem Koppelabschnitt (42) angeordnet ist, der lösbar an dem Gehäuse (14, 16), vorzugsweise an einem Kopfbereich (14) desselben, angeordnet ist.
3. Diagnosekamera nach Aspekt 1 oder 2, bei der ein freier Endabschnitt (46) des Abstandhalterabschnittes (44) eine Auflagefläche vorgibt, die im wesentlichen senkrecht auf einer Blickrichtung (42) der Optik (18) steht.
4. Diagnosekamera nach einem der Aspekte 1 bis 3, bei der ein freier Endabschnitt (46) des Abstandhalterabschnittes (44) als zumindest im Wesentlichen umlaufende, vorzugsweise in Umfangsrichtung geschlossene, Hülse gestaltet ist.
5. Diagnosekamera nach einem der vorhergehenden Aspekte, bei der der Koppelabschnitt (42) als zumindest im Wesentlichen umlaufende, vorzugsweise geschlossene, Hülse gestaltet ist.
6. Diagnosekamera nach einem der Aspekte 2 bis 5, bei der der Abstandshalter (44) und der Koppelabschnitt (42) hülsenförmig sind und zusammen einen durchgehenden, vorzugsweise um 90 Grad abgewinkelten, Kanal begrenzen.
7. Diagnosekamera nach Aspekt 6, bei der der Kanal an einer Innenseite zumindest im Bereich des Abstandshalterabschnittes (44) mit einer lichtabsorbierenden oder Licht streuenden Oberfläche (54) versehen ist.
8. Diagnosekamera nach Aspekt 7 bei der der Kanal im Bereich des Abstandshalterabschnittes (44) an der Innenseite mit einer vergrößerten Rauhigkeit, vorzugsweise mit Rillen, versehen ist.
9. Diagnosekamera nach einem der vorhergehenden Aspekte, bei der sich der Abstandshalterabschnitt (44) zum freien Ende hin erweitert, vorzugsweise konisch oder pyramidenförmig erweitert.
10. Diagnosekamera nach einem der Aspekte 2 bis 9, bei der der Koppelabschnitt (42) und der Abstandshalterabschnitt (44) einstückig, insbesondere im Kunststoffspritzgussverfahren, hergestellt sind.
11. Diagnosekamera nach einem der Aspekte I bis 10, bei der der Abstandshalterabschnitt (44)und vorzugsweise auch mit diesem einstückig verbundene Teile eines Koppelabschnittes (42) und nochmals vorzugsweise auch das Gehäuse (14, 16) aus einem sterilisierbaren und/oder desinfizierbaren Kunststoff, beispielsweise einem Polypropylen, hergestellt ist.
12. Diagnosekamera nach einem der Aspekte 2 bis 11, bei der der Koppelabschnitt (42) derart auf den Kopfbereich (14) des Gehäuses (14, 16) abgestimmt ist, dass er in einer aufgeschobenenen Arbeitsstellung formschlüssig am Kopfbereich (14) festgelegt ist.
13. Diagnosekamera nach einem der Aspekte 1 bis 12, dadurch gekennzeichnet, dass der freie Endabschnitt (46) eine transparente Endplatte (55) trägt.
14. Diagnosekamera nach Anspruch 13 in Verbindung mit Anspruch 4, dadurch gekennzeichnet, dass die Endplatte (55) dicht mit der Hülse verbunden ist.

## Patentansprüche

1. Abstandshalter-Aufsatz für eine Diagnosekamera, insbesondere für medizinische und dentale Zwecke, die ein Gehäuse (14, 16), eine Optik (18) und einen Bildaufnehmer (20) aufweist, die in dem Gehäuse (14, 16) untergebracht sind, **dadurch gekennzeichnet, dass** das er einen Abstandshalterabschnitt (44) aufweist, der auf einem Koppelabschnitt (42) angeordnet ist, der lösbar an dem Gehäuse (14, 16), vorzugsweise an einem Kopfbereich (14) desselben, angeordnet werden kann.

2. Abstandshalter-Aufsatz nach Anspruch 1, **dadurch gekennzeichnet, dass** ein freier Endabschnitt (46) des Abstandhalterabschnittes (44) eine Auflagefläche vorgibt, die im wesentlichen senkrecht auf einer Längsachse des Abstandshalterabschnittes (44) steht.

3. Abstandshalter-Aufsatz nach Anspruch 1 oder 2, **dadurch gekennzeichnet, dass** ein freier Endabschnitt (46) des Abstandhalterabschnittes (44) als zumindest im Wesentlichen umlaufende, vorzugsweise in Umfangsrichtung geschlossene, Hülse gestaltet ist.

4. Abstandshalter-Aufsatz nach einem der Ansprüche 1 bis 3, **dadurch gekennzeichnet, dass** der Koppelabschnitt (42) a-s zumindest im Wesentlichen umlaufende, vorzugsweise geschlossene, Hülse gestaltet ist.

5. Abstandshalter-Aufsatz nach einem der Ansprüche 2 bis 4, **dadurch gekennzeichnet, dass** der Abstandshalterab-schnitt (44) und der Koppelabschnitt (42) hülsenförmig sind und zusammen einen durchgehenden, vorzugsweise um 90 Grad abgewinkelten, Kanal begrenzen.

6. Abstandshalter-Aufsatz nach Anspruch 5, **dadurch gekennzeichnet, dass** der Kanal an einer Innenseite zumindest im Bereich des Abstandshalterabschnittes (44) mit einer Licht absorbierenden oder licht streuenden Oberfläche (54) versehen ist.

7. Abstandshalter-Aufsatz nach Anspruch 6 **dadurch gekennzeichnet, dass** der Kanal im Bereich des Abstandshalterabschnittes (44) an der Innenseite mit einer vergrößerten Rauhigkeit, vorzugsweise mit Rillen, versehen ist.

8. Abstandshalter-Aufsatz nach einem der Ansprüche 1 bis 7, **dadurch gekennzeichnet, dass** sich der Abstandshalterabschnitt (44) zum freien Ende hin erweitert, vorzugsweise konisch oder pyramidenförmig erweitert.

9. Abstandshalter-Aufsatz nach einem der Ansprüche 1 bis 8, **dadurch gekennzeichnet, dass** der Koppelabschnitt (42) und der Abstandshalterabschnitt (44) einstückig,^ insbesondere im Kunststoffspritzgussverfahren, hergestellt sind.

10. Abstandshalter-Aufsatz nach einem der Ansprüche 1 bis 9, **dadurch gekennzeichnet, dass** der Abstandshalterabschnitt (44)und vorzugsweise auch mit diesem einstückig verbundene Teile des Koppelabschnittes (42) aus einem sterilisierbaren und/oder desinfizierbaren Kunststoff, beispielsweise einem Polypropylen, hergestellt ist.

11. Abstandshalter-Aufsatz nach einem der Ansprüche 1 bis 10, **dadurch gekennzeichnet, dass** der Koppelabschnitt (42) derart auf den Kopfbereich (14) des Gehäuses (14, 16) abgestimmt ist, dass er in einer aufgeschobenenen Arbeitsstellung formschlüssig am Kopfbereich (14) festgelegt ist.

12. Abstandshalter-Aufsatz nach einem der Ansprüche 2 bis 11, **dadurch gekennzeichnet, dass** der freie Endabschnitt (46) eine transparente Endplatte (55) trägt.

13. Abstandshalter nach Anspruch 12 in Verbindung mit Anspruch 3, **dadurch gekennzeichnet, dass** die Endplatte (55) dicht mit der Hülse verbunden ist.
